# EUROPEAN PATENT APPLICATION

(11) **EP 3 919 113 A1**
(43) Date of publication of application: **08.12.2021**
(21) Application number: 20771129.2
(22) Date of filing: 03.02.2020
(51) Int. Cl.: A61M 39/02, A61M 39/10, A61M 39/22

(54) **MEDICAL INSTRUMENT**

(30) Priority: 08.03.2019 JP 2019042171
(71) Applicant: TERUMO Kabushiki Kaisha, Tokyo 151-0072 (JP)
(72) Inventor: KAKINOKI, Toshihiko, Tokyo 163-1450 (JP)
(74) Representative: Casalonga
(86) International application number: PCT/JP2020/003978
(87) International publication number: WO 2020/183978

(57) **Abstract**

A medical device according to the disclosure includes: two members that are made of materials having different flexural moduli and are connected to each other, in which a soft member made of a material having a low flexural modulus of the two members includes a cylindrical portion, a hard member made of a material having a high flexural modulus of the two members includes a cylindrical support portion that supports the cylindrical portion, and the cylindrical support portion includes an inner surface support portion that supports an inner surface of the cylindrical portion, and an outer surface support portion that supports an outer surface of the cylindrical portion.

## Description

### Technical Field

The present disclosure relates to a medical device.

### Background Art

In related arts, there is a medical device configured by connecting a plurality of members. PTL 1 describes a medical device of the above type.

A medical stopcock as the medical device described in PTL 1 includes: a stopcock main body including a cylindrical chamber portion and a plurality of branch pipes; and a valve body that is installed in the chamber portion in a state of being rotatable in a direction around an axis of the chamber portion and in which a groove portion for communicating an optional flow paths among flow paths of the plurality of branch pipes is formed on an outer peripheral surface. A connecting portion for connecting to another member in a predetermined branch pipe of PTL 1 is configured with a member separate from another portion configuring the stopcock main body. PTL 1 describes that the stopcock main body is made of polycarbonate, and the above-described connection portion, which is the separate member, is made of polypropylene. The connecting portion made of polypropylene described in PTL 1 includes an engagement cylindrical portion and a cylindrical proximal end portion located outside the engagement cylindrical portion. Further, a coupling portion of the stopcock main body in PTL 1 is inserted and connected between the engagement cylindrical portion and the proximal end portion of the connecting portion.

### Citation List

### Patent Literature

PTL 1: JP-A-2007-143813

### Summary of Invention

### Technical Problem

In the medical stopcock described in PTL 1, the cylindrical coupling portion of the stopcock main body made of polycarbonate that is relatively hard to deform is connected to the connecting portion in a state of being sandwiched between the engagement cylindrical portion and the cylindrical proximal end portion of the connection portion made of polypropylene that is relatively easy to deform. Therefore, in the medical stopcock of PTL 1, if an external force (hereinafter, referred to as "bending external force") that bends one of the stopcock main body and the connection portion to the other is applied, the connecting portion is deformed and falls off from the stopcock main body, the connection between the two may be released.

An object of the present disclosure is to provide a medical device having a configuration in which connection is not easily released even when a bending external force acts on two members connected to each other.

### Solution to Problem

A medical device according to the disclosure includes: two members that are made of materials having different flexural moduli and are connected to each other, in which a soft member made of a material having a low flexural modulus of the two members includes a cylindrical portion, a hard member made of a material having a high flexural modulus of the two members includes a cylindrical support portion that supports the cylindrical portion, and the cylindrical support portion includes an inner surface support portion that supports an inner surface of the cylindrical portion, and an outer surface support portion that supports an outer surface of the cylindrical portion.

As an embodiment of the present disclosure, the soft member and the hard member are rotatably connected to each other.

As an embodiment of the present disclosure, the hard member includes a male connector portion that can be inserted into a female connector portion of another medical device.

As an embodiment of the present disclosure, a hollow portion of the male connector portion of the hard member communicates with a hollow portion of the cylindrical portion of the soft member.

As an embodiment of the present disclosure, the hard member includes a locking portion that is located on a radially outer side of the male connector portion and can be locked to another medical device.

As an embodiment of the present disclosure, the locking portion is a locking cylindrical portion in which a female screw portion capable of being screwed to another medical device is formed on an inner surface.

As an embodiment of the present disclosure, the soft member includes a female connector portion into which a male connector portion of still another medical device is inserted.

As an embodiment of the present disclosure, the inner surface support portion is an inner cylindrical portion inserted into the cylindrical portion, and an outer surface of the inner cylindrical portion supports an inner surface of the cylindrical portion.

As an embodiment of the present disclosure, the outer surface support portion is an outer cylindrical portion surrounding a radially outer side of the cylindrical portion, and an inner surface of the outer cylindrical portion supports an outer surface of the cylindrical portion.

### Advantageous Effect of Invention

According to the present disclosure, a medical device having a configuration in which connection is not easily released even when a bending external force acts on two members connected to each other can be provided.

### Brief Description of Drawings

[Fig. 1] Fig. 1 is a perspective view showing a medical device according to an embodiment of the present disclosure.
[Fig. 2] Fig. 2 is an exploded perspective view of the medical device shown in Fig. 1.
[Fig. 3] Fig. 3 is a top view of the medical device shown in Fig. 1.
[Fig. 4] Fig. 4 is a cross-sectional view taken along a line I-I in Fig. 3.
[Fig. 5] Fig. 5 is a cross-sectional view taken along a line II-II in Fig. 3.
[Fig. 6] Fig. 6 is a cross-sectional view showing a state in which a device main body and a connection member shown in Fig. 1 are separated from each other in the same cross-sectional view as Fig. 4.
[Fig. 7] Fig. 7 is a cross-sectional view showing a state in which two medical devices shown in Fig. 1 are connected in the same cross-sectional view as Fig. 4.
[Fig. 8] Fig. 8 is a diagram showing a modification of a cylindrical support portion shown in Fig. 4 and the like.
[Fig. 9] Fig. 9 is a diagram showing an infusion set including the medical device shown in Fig. 1.

### Description of Embodiments

Hereinafter, an embodiment of a medical device according to the present disclosure will be described as an example with reference to drawings. In the drawings, common members and portions are denoted by the same reference numerals.

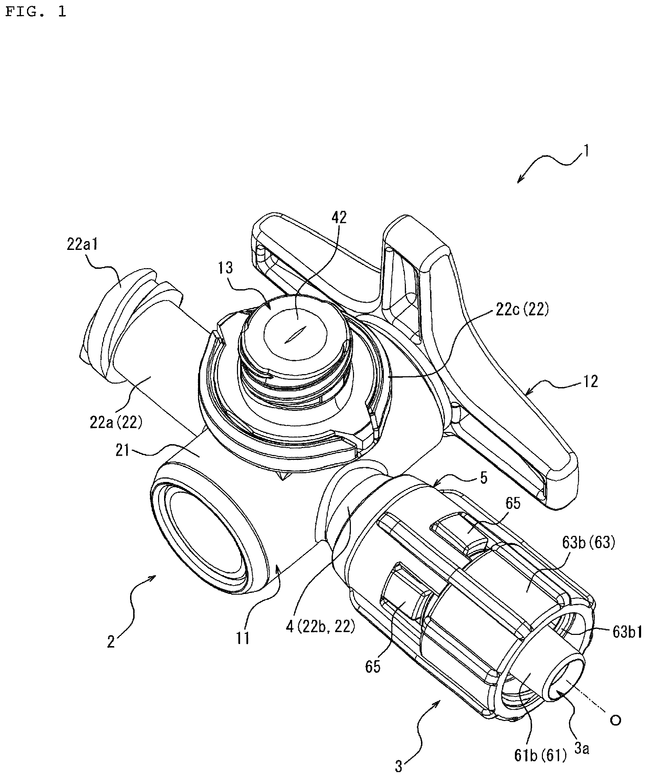

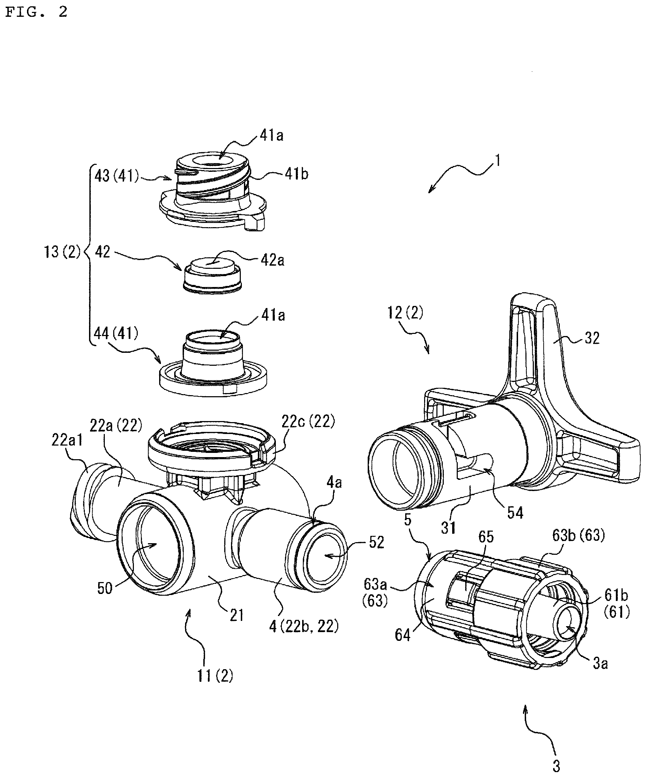

Fig. 1 is a perspective view showing a medical device 1 according to an embodiment of the present disclosure. Fig. 2 is an exploded perspective view of the medical device 1. The medical device 1 shown in Figs. 1 and 2 is a three-way stopcock. Although in the present embodiment, a three-way stopcock will be described as an example of the medical device, the medical device according to the present disclosure is not limited to the three-way stopcock shown here. The medical device according to the present disclosure may be another medical connector different from the three-way stopcock, such as a T-shaped needleless connector.

As shown in Figs. 1 and 2, the medical device 1 includes a device main body 2 and a connection member 3. The device main body 2 includes a holder member 11, a cock member 12, and a mixed injection member 13.

The medical device 1 includes two members that are made of materials having different flexural moduli and are connected to each other. Although details will be described later, in the medical device 1 according to the present embodiment, at least the connection member 3 and the holder member 11 described above are made of materials having different flexural moduli and connected to each other.

The holder member 11 is made of a material having a lower flexural modulus than the connection member 3. In other words, the connection member 3 is made of a material having a higher flexural modulus than the holder member 11. Here, the "flexural modulus" means a bending property measured by a method defined in JIS K7171: 2016. Hereinafter, the connection member 3 may be referred to as a "hard member", and the holder member 11 may be referred to as a "soft member".

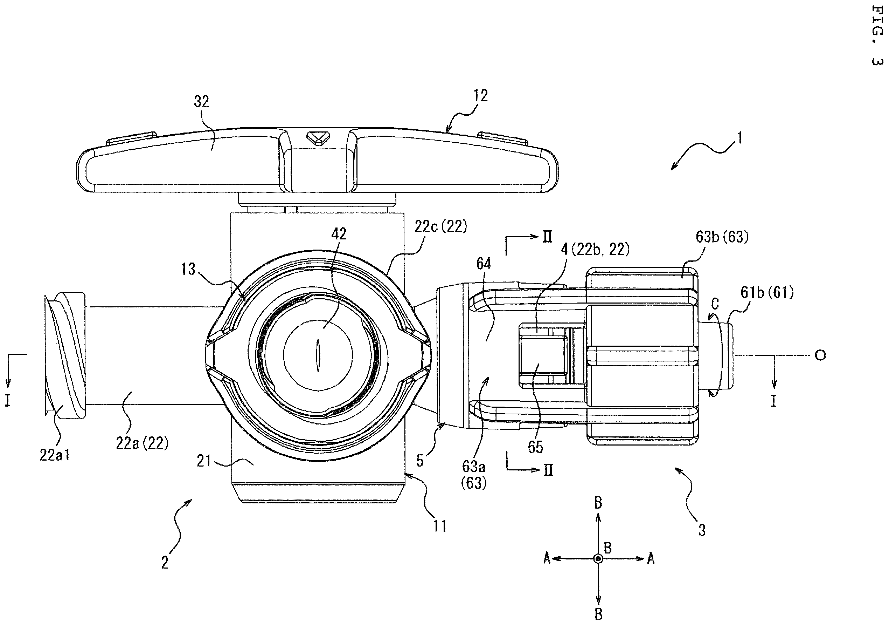

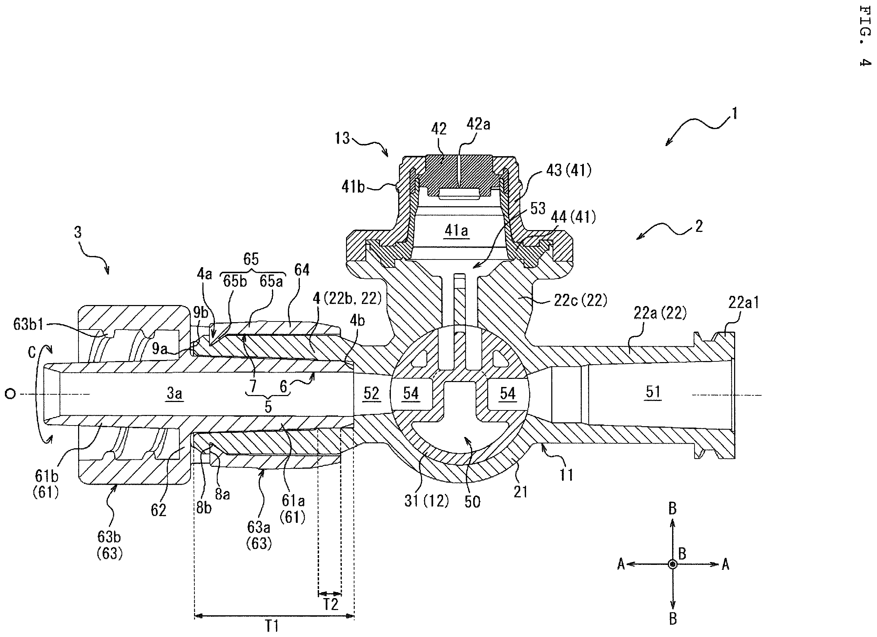

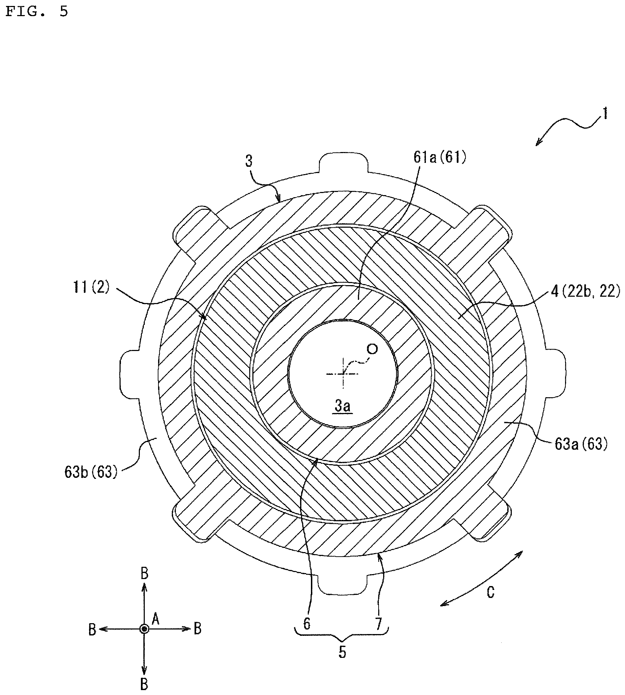

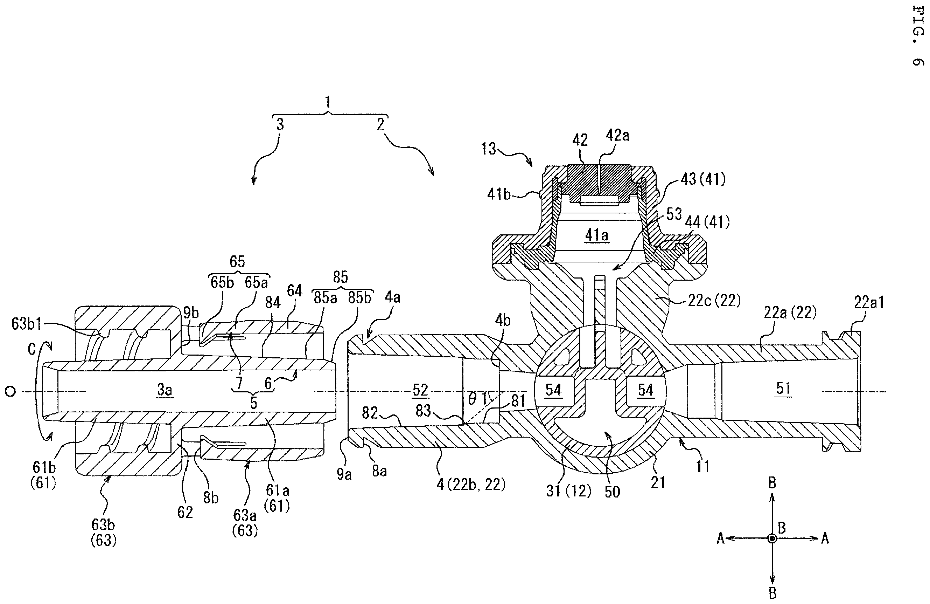

Fig. 3 is a top view of the medical device 1. Fig. 4 is a cross-sectional view of the medical device 1 taken along a line I-I in Fig. 3. Fig. 5 is a cross-sectional view of the medical device 1 taken along a line II-II in Fig. 3. Fig. 6 is a cross-sectional view showing a state in which the device main body 2 and the connection member 3 are separated from each other. Fig. 6 shows the same cross-sectional view as Fig. 4. As shown in Figs. 3 to 6, the holder member 11 as the soft member includes a cylindrical portion 4. The connection member 3 as the hard member includes a cylindrical support portion 5 that supports the cylindrical portion 4 of the holder member 11 as the soft member. The cylindrical support portion 5 includes an inner surface support portion 6 that supports an inner surface of the cylindrical portion 4, and an outer surface support portion 7 that supports an outer surface of the cylindrical portion 4.

With such a configuration, the cylindrical portion 4 of the holder member 11 as the soft member is less likely to be deformed by an external force (hereinafter, simply referred to as a "bending external force") that bends one of the holder member 11 as the soft member and the connection member 3 as the hard member, which are connected to each other, to the other. Therefore, the connection between the holder member 11 and the connection member 3 is not easily released. Specifically, although the cylindrical portion 4 of the holder member 11 as the soft member tends to be deformed by the above-described bending external force, the cylindrical support portion 5 of the connection member 3 as the hard member comes into contact with the inner surface and the outer surface of the cylindrical portion 4, thereby restricting a deformation of the cylindrical portion 4. Accordingly, the cylindrical portion 4 of the holder member 11 as the soft member is less likely to be deformed by the above-described bending external force.

The above-described "bending external force" according to the present embodiment means an external force that causes one of the holder member 11 and the connection member 3 to rotate relative to the other in a plane including a central axis O (see Fig. 4 and the like) of the cylindrical portion 4.

Next, further details of the medical device 1 according to the present embodiment will be described with reference to Figs. 1 to 6. Hereinafter, in the medical device 1 in a state where the device main body 2 and the connection member 3 are connected to each other, an axial direction of the cylindrical portion 4 of the holder member 11 to be described later and an axial direction of an inner cylindrical portion 61 of the connection member 3 to be described later will be simply referred to as an "axial direction A". Further, in the medical device 1 in a state where the device main body 2 and the connection member 3 are connected to each other, a radial direction of the cylindrical portion 4 of the holder member 11 to be described later and a radial direction of the inner cylindrical portion 61 of the connection member 3 to be described later will be simply referred to as an "radial direction B". Further, in the medical device 1 in the state where the device main body 2 and the connection member 3 are connected to each other, a circumferential direction of the cylindrical portion 4 of the holder member 11 to be described later and a circumferential direction of the inner cylindrical portion 61 of the connection member 3 to be described later will be simply referred to as a "circumferential direction C".

### [Device Main Body 2]

### <Holder Member 11>

The holder member 11 includes a holder main body 21 and a plurality of port portions 22. The plurality of port portions 22 protrude outward from the holder main body 21.

The holder main body 21 has a substantially cylindrical outer shape. Therefore, the holder main body 21 defines a substantially cylindrical hollow portion 50 therein. The cock member 12, which will be described later, is accommodated in the hollow portion 50. The port portions 22 protrude from the holder main body 21. The port portions 22 define a flow path communicating with the hollow portion 50 of the holder main body 21.

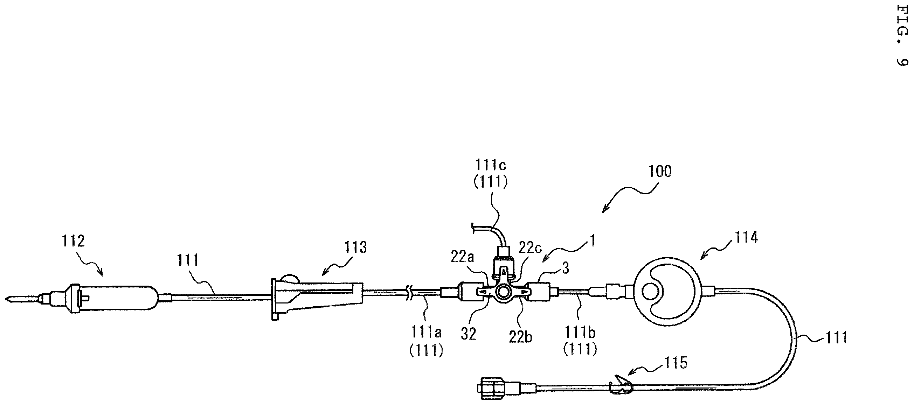

The plurality of port portions 22 according to the present embodiment are constituted of a first port portion 22a, a second port portion 22b, and a third port portion 22c. Each of the port portions 22 is formed integrally with the holder main body 21 and extends in the radial direction of the holder main body 21. The first port portion 22a is a cylindrical portion that defines a first flow path 51 that communicates with the hollow portion 50, and is connected to another medical device such as a medical tube 111 (see Fig. 9). The second port portion 22b is the cylindrical portion 4 that defines a second flow path 52 that communicates with the hollow portion 50, and is connected to another medical device such as the medical tube 111 (see Fig. 9), via the connection member 3 described later. The third port portion 22c defines a third flow path 53 communicating with the hollow portion 50, and is connected to another medical device such as a syringe and the medical tube 111 which have a cylindrical male connector portion at a distal portion (see Fig. 9), via the mixed injection member 13 described later.

Hereinafter, for convenience of description, another medical device connected to the first port portion 22a will be described as a "first other medical device". Another medical device connected to the second port portion 22b via the connection member 3 will be described as a "second other medical device". Further, another medical device connected to the third port portion 22c via the mixed injection member 13 will be described as a "third other medical device".

The first port portion 22a according to the present embodiment is a cylindrical portion integrally formed with the holder main body 21. In a state where the first other medical device is connected to the first port portion 22a, a liquid such as a drug solution flows into the hollow portion 50 of the holder main body 21 from the first other medical device through the first flow path 51 of the first port portion 22a. Therefore, the first port portion 22a according to the present embodiment constitutes a port portion on a flow path upstream side of the medical device 1.

More specifically, the first port portion 22a according to the present embodiment is a female connector portion into which a cylindrical male connector portion of another medical device is inserted. That is, another medical device is connected to the first port portion 22a by inserting the cylindrical male connector portion of another medical device from a distal opening. A male screw portion 22a1 is formed on an outer surface of the first port portion 22a. The male screw portion 22a1 is configured to be capable of being screwed to a female screw portion of a lock-type male connector portion conforming to ISO 80369-7 issued in 2016.

The second port portion 22b according to the present embodiment is the cylindrical portion 4 integrally formed with the holder main body 21 at a position opposite to the first port portion 22a with the holder main body 21 sandwiched therebetween. Although details will be described later, the connection member 3 is connected to the second port portion 22b. The second flow path 52 of the second port portion 22b communicates with a flow path 3a defined by the connection member 3. A second other medical device is connected to the connection member 3. In a state in which the second other medical device is connected to the second port portion 22b via the connection member 3, the liquid such as the drug solution flows out from the medical device 1 to the second other medical device through the second flow path 52 of the second port portion 22b and the flow path 3a of the connection member 3. Therefore, the second port portion 22b and the connection member 3 described later according to the present embodiment constitute a port portion on a flow path downstream side of the medical device 1.

As shown in Fig. 6, an inner peripheral surface of the cylindrical portion 4 constituting the second port portion 22b includes a proximal inner peripheral surface 81 located on a proximal side, a distal inner peripheral surface 82 located on a distal side and extending to a distal end, and an intermediate inner peripheral surface 83 located between the proximal inner peripheral surface 81 and the distal inner peripheral surface 82 in the axial direction A.

As shown in Fig. 6, each of the proximal inner peripheral surface 81, the distal inner peripheral surface 82, and the intermediate inner peripheral surface 83 is a tapered surface whose diameter increases from the proximal side (a right side in Fig. 6) toward the distal side (a left side in Fig. 6). However, for taper angles with respect to the central axis O of the cylindrical portion 4 constituting the second port portion 22b, a taper angle of the intermediate inner peripheral surface 83 is larger than a taper angle of each of the proximal inner peripheral surface 81 and the distal inner peripheral surface 82. In Fig. 6, the taper angle of the intermediate inner peripheral surface 83 is illustrated as "θ1", and the taper angles of the proximal inner peripheral surface 81 and the distal inner peripheral surface 82 also mean angles at similar positions.

As described above, the proximal inner peripheral surface 81 and the distal inner peripheral surface 82 according to the present embodiment are configured with tapered surfaces, but the present invention is not limited to this configuration and the proximal inner peripheral surface 81 and the distal inner peripheral surface 82 may be configured as inner peripheral surfaces having a uniform inner diameter in the axial direction A. In the present embodiment, the taper angle of the proximal inner peripheral surface 81 and the taper angle of the distal inner peripheral surface 82 are substantially equal to each other, but the taper angles may be different from each other.

As shown in Fig. 4, an outer peripheral surface of a coupling inner cylindrical portion 61a of the connection member 3, which will be described later, fitted into the cylindrical portion 4 constituting the second port portion 22b is in close contact with a proximal inner peripheral surface 81 of the cylindrical portion 4. Details of a distal outer peripheral surface 85 of the outer peripheral surface of the coupling inner cylindrical portion 61a, which is in close contact with the proximal inner peripheral surface 81 described above, will be described later.

As shown in Fig. 4, an insertion restricting portion 4b is provided on the inner surface of the cylindrical portion 4 constituting the second port portion 22b. The insertion restricting portion 4b restricts further insertion of the connection member 3 by being in contact with a distal surface of the coupling inner cylindrical portion 61a of the connection member 3, which will be described later. As shown in Fig. 6, the proximal inner peripheral surface 81, the intermediate inner peripheral surface 83, and the distal inner peripheral surface 82 are located closer to the distal side of the cylindrical portion 4 relative to the insertion restricting portion 4b in the axial direction A.

As shown in Fig. 6 and the like, an annular groove 4a extending in the circumferential direction C is formed on the outer surface of the cylindrical portion 4 constituting the second port portion 22b. More specifically, the annular groove 4a according to the present embodiment is formed on the outer surface of the cylindrical portion 4 in a region in the axial direction A where the distal inner peripheral surface 82 of the cylindrical portion 4 is formed. As shown in Fig. 4, a claw portion 65 of the connection member 3 is fitted into the annular groove 4a. Details of the claw portion 65 will be described below.

The third port portion 22c according to the present embodiment is provided on a peripheral surface of the holder main body 21 at a position different from the positions where the first port portion 22a and the second port portion 22b are provided. More specifically, the third port portion 22c according to the present embodiment is integrally formed with the holder main body 21 at a position shifted by about 90 degrees from the position where the first port portion 22a and the second port portion 22b are provided in the circumferential direction of the peripheral surface of the holder main body 21. The third port portion 22c constitutes a mixed injection port portion. The mixed injection port portion is, for example, a port portion used when a liquid such as a drug solution different from the liquid flowing into the medical device 1 from the first flow path 51 of the first port portion 22a flows into the medical device 1.

The mixed injection member 13 to which a cylindrical male connector portion of the third other medical device can be connected is attached to a distal portion of the third port portion 22c according to the present embodiment. The mixed injection member 13 according to the present embodiment includes a cap member 41 and an elastic valve body 42. Details of the mixed injection member 13 will be described later.

Examples of the material of the holder member 11 include various resin materials such as polyolefins such as polyethylene, polypropylene, and ethylene-propylene copolymer; ethylene-vinyl acetate copolymer (EVA); polyvinyl chloride; polyvinylidene chloride; polystyrene; polyamide; polyimide; polyamideimide; polycarbonate; poly-(4-methylpentene-1); ionomer; acrylic resin; polymethyl methacrylate; acrylonitrile-butadiene-styrene copolymer (ABS resin); acrylonitrile-styrene copolymer (AS resin); butadiene-styrene copolymer; polyesters such as polyethylene terephthalate (PET), polybutylene terephthalate (PBT), and polycyclohexane terephthalate (PCT); polyether; polyether ketone (PEK); polyether ether ketone (PEEK); polyether imide; polyacetal (POM); polyphenylene oxide; modified polyphenylene oxide; polysulfone; polyethersulfone; polyphenylene sulfide; polyarylate; aromatic polyester (liquid crystal polymer); polytetrafluoroethylene; polyvinylidene fluoride; and other fluorine-based resins.

### <Cock Member 12>

Next, the cock member 12 accommodated in the hollow portion 50 defined by the holder main body 21 will be described. The cock member 12 is rotatable about a central axis of the holder main body 21 in a state in which the cock member 12 is accommodated in the hollow portion 50 of the holder main body 21. For example, the cock member 12 rotates relative to the holder member 11 while sliding on an inner surface of the holder main body 21 that defines the hollow portion 50. In this way, when the cock member 12 rotates in the holder main body 21, a connection state between the flow paths (the first flow path 51, the second flow path 52, and the third flow path 53 according to the present embodiment) respectively defined by the plurality of port portions 22 (the first port portion 22a, the second port portion 22b, and the third port portion 22c according to the present embodiment) changes. Here, the connection state between the flow paths changes when the opening and closing states of the flow paths are switched by the rotating cock member 12. That is, the connection state between the flow paths changes by switching between a closed state in which the flow paths are closed by the cock member 12 and an open state in which the flow paths are not closed by the cock member 12 by the rotation of the cock member 12.

As shown in Fig. 2 and the like, the cock member 12 according to the present embodiment includes a cock main body 31 that is accommodated inside the holder main body 21 of the holder member 11, and a handle portion 32 that is provided at one end of the cock main body 31 and located outside the holder member 11.

The cock main body 31 is rotatable about a central axis of the holder main body 21 in a state in which the cock main body 31 is accommodated in the hollow portion 50 of the holder main body 21. The cock main body 31 defines a connection flow path 54 that connects the flow paths (the first flow path 51, the second flow path 52, and the third flow path 53 according to the present embodiment) of the plurality of port portions 22 (the first port portion 22a, the second port portion 22b, and the third port portion 22c in the present embodiment) of the holder member 11. When the cock main body 31 rotates with respect to the holder member 11 in the hollow portion 50, the connection state between the connection flow path 54 of the cock main body 31 and the flow paths defined by the plurality of port portions 22 of the holder member 11 can be changed.

As shown in Fig. 2 and the like, the cock main body 31 according to the present embodiment has a substantially cylindrical outer shape that is accommodated in the hollow portion 50 of the holder main body 21. An outside diameter of the cock main body 31 is substantially equal to an inside diameter of the inner surface of the holder main body 21 that defines the hollow portion 50. Therefore, the cock main body 31 according to the present embodiment rotates relative to the holder member 11 while sliding on the inner surface of the holder main body 21 in a state in which the cock main body 31 is accommodated in the hollow portion 50.

The handle portion 32 is a portion that is gripped and operated by the user. The handle portion 32 is formed integrally with the cock main body 31. Therefore, by rotating the handle portion 32 about the central axis of the holder main body 21, the cock main body 31 located in the hollow portion 50 can be rotated about the central axis of the holder main body 21.

### <Mixed Injection Member 13>

The mixed injection member 13 is attached to the distal portion of the third port portion 22c of the holder member 11. The mixed injection member 13 according to the present embodiment includes a cap member 41 that defines an insertion opening 41a into which the cylindrical male connector portion of the third other medical device can be inserted, and an elastic valve body 42 that has a slit 42a and closes the insertion opening 41a. The male connector portion of the third other medical device is inserted into the medical device 1 from the insertion opening 41a through the slit 42a of the elastic valve body 42. In this way, the third port portion 22c of the holder member 11 can be connected to the third other medical device via the mixed injection member 13.

More specifically, the mixed injection member 13 according to the present embodiment is a female connector portion into which the cylindrical male connector portion of the third other medical device is inserted. That is, the other medical device is connected to the mixed injection member 13 by inserting the cylindrical male connector portion of the other medical device from an insertion opening 41a. A male screw portion 41b is formed on the outer surface of the cap member 41 of the mixed injection member 13. The male screw portion 41b is configured to be capable of being screwed with a female screw portion of a lock-type male connector portion conforming to ISO 80369-7 issued in 2016.

Examples of the material of the cap member 41 include materials similar to the various materials that can be used as the material of the holder member 11 described above. The material constituting the cap member 41 may be the same as or different from the material constituting the holder member 11.

The elastic valve body 42 is formed by molding and is formed to be elastically deformable. Examples of the material of the elastic valve body 42 include various rubber materials such as natural rubber, isoprene rubber, butadiene rubber, styrene-butadiene rubber, nitrile rubber, chloroprene rubber, butyl rubber, acrylic rubber, ethylene-propylene rubber, hydrin rubber, urethane rubber, silicone rubber, and fluorine rubber, and various thermoplastic elastomers such as styrene-based, polyolefin-based, polyvinyl chloride-based, polyurethane-based, polyester-based, polyamide-based, polybutadiene-based, transpolyisoprene-based, fluorine rubber-based, and chlorinated polyethylene-based thermoplastic elastomers, and one of these may be used alone, or two or more of these may be used in combination.

Although the cap member 41 of the mixed injection member 13 according to the present embodiment is configured with a top surface cap 43 and a bottom surface cap 44 that sandwich and compress the circular flat elastic valve body 42 from a top surface (a surface on a side exposed outward) and a bottom surface that is a surface on an opposite side of the top surface, the present invention is not limited to this configuration. The cap member may have, for example, a configuration in which the third port portion and the bottom surface cap are integrated, that is, a configuration in which the elastic valve body is sandwiched and compressed by the third port portion of the holder member and the top surface cap.

### [Connection Member 3]

The connection member 3 is made of a single material. As shown in Fig. 4, the connection member 3 includes a substantially cylindrical inner cylindrical portion 61 that defines the flow path 3a, an annular flange portion 62 that protrudes outward in the radial direction B from an outer surface of the inner cylindrical portion 61, and a substantially cylindrical outer cylindrical portion 63 that protrudes from the annular flange portion 62 in the axial direction A.

The inner cylindrical portion 61 includes the coupling inner cylindrical portion 61a that protrudes from the annular flange portion 62 toward one end side (a right side in Fig. 4) in the axial direction A and is inserted into the cylindrical portion 4 constituting the second port portion 22b of the holder member 11 described above, and a distal inner cylindrical portion 61b that protrudes from the annular flange portion 62 toward the other end side (a left side in Fig. 4) in the axial direction A.

As shown in Fig. 6, the outer peripheral surface of the coupling inner cylindrical portion 61a of the inner cylindrical portion 61 includes a proximal outer peripheral surface 84 continuous with the annular flange portion 62 and a distal outer peripheral surface 85 located on the distal side and extending to the distal end.

As shown in Fig. 6, each of the proximal outer peripheral surface 84 and a distal outer peripheral surface 85 is tapered surface whose diameter decreases from the proximal side toward the distal side (from the left side to the right side in Fig. 4).

Although as described above, the proximal outer peripheral surface 84 and the distal outer peripheral surface 85 according to the present embodiment are configured with tapered surfaces, the present invention is not limited to this configuration, and may be configured as inner peripheral surfaces having a uniform inner diameter in the axial direction A. Each of the proximal outer peripheral surface 84 and the distal outer peripheral surface 85 may have a uniform taper angle regardless of a position in the axial direction A. In this case, the taper angles of the proximal outer peripheral surface 84 and the distal outer peripheral surface 85 may be substantially equal to each other or may be different from each other. Although in the present embodiment, the taper angle of the proximal outer peripheral surface 84 is different from a taper angle of a distal root portion surface 85a (described later) of the distal outer peripheral surface 85, the taper angles may be substantially equal to each other.

As shown in Fig. 6, the distal outer peripheral surface 85 according to the present embodiment is configured with two tapered surfaces having different taper angles. Specifically, the distal outer peripheral surface 85 according to the present embodiment includes a distal root portion surface 85a continuous with the proximal outer peripheral surface 84, and a distal portion surface 85b having a taper angle larger than that of the distal root portion surface 85a and extending to the distal end. Although the distal outer peripheral surface 85 may be formed of one cylindrical surface or one tapered surface, it is preferable that the distal outer peripheral surface 85 is formed of a plurality of outer peripheral surfaces having different taper angles as in the present embodiment. In this way, when the connection member 3 is connected to the holder member 11, the coupling inner cylindrical portion 61a can be smoothly fitted to the cylindrical portion 4 constituting the second port portion 22b of the holder member 11.

As shown in Fig. 4, the inner peripheral surface of the cylindrical portion 4 constituting the second port portion 22b of the holder member 11 and externally fitted to the coupling inner cylindrical portion 61a is in close contact with the distal outer peripheral surface 85 of the coupling inner cylindrical portion 61a. More specifically, the distal outer peripheral surface 85 of the coupling inner cylindrical portion 61a is in close contact with the proximal inner peripheral surface 81 of the cylindrical portion 4 of the holder member 11.

In this way, the coupling inner cylindrical portion 61a of the connection member 3 is inserted into the cylindrical portion 4 of the holder member 11, and an insertion region T1 in the axial direction A in which the cylindrical portion 4 and the coupling inner cylindrical portion 61a overlap each other in the radial direction B is formed (see Fig. 4). However, as shown in Fig. 4, a contact region T2 where the inner peripheral surface of the cylindrical portion 4 and the outer peripheral surface of the coupling inner cylindrical portion 61a are in close contact with each other is only a part of the above-described insertion region T1. In this way, compared to a configuration in which the contact region T2 is the entire insertion region T1, it is possible to reduce a sliding resistance when the holder member 11 and the connection member 3 relatively rotate around the central axis O of the cylindrical portion 4.

In the present embodiment, the taper angle of the distal root portion surface 85a relative to the axial direction A is smaller than the taper angle (see "θ1" in Fig. 6) of the intermediate inner peripheral surface 83 relative to the axial direction A. In this way, it is easy to implement a configuration in which the distal root portion surface 85a comes into contact with the proximal inner peripheral surface 81 without coming into contact with the intermediate inner peripheral surface 83. Further, the taper angle of the distal root portion surface 85a relative to the axial direction A is larger than the taper angle of the proximal inner peripheral surface 81 relative to the axial direction A.

The contact region T2 of the present embodiment is formed over an entire region in the circumferential direction C of the cylindrical portion 4 and the coupling inner cylindrical portion 61. That is, the proximal inner peripheral surface 81 (see Fig. 6) of the inner peripheral surface of the cylindrical portion 4 is in contact with the distal outer peripheral surface 85 (see Fig. 6) of the outer peripheral surface of the coupling inner cylindrical portion 61a over the entire region in the circumferential direction C. Accordingly, the hollow portion of the cylindrical portion 4 and the flow path 3a as the hollow portion defined by the connection member 3 are liquid-tightly connected to each other. In this way, sealing property of a connection location may be ensured by bringing the outer surface of the coupling inner cylindrical portion 61 of the connection member 3 into contact with the inner surface of the cylindrical portion 4 of the holder member 11, but the sealing property may be ensured by, for example, interposing another member such as an O-ring between the outer surface of the coupling inner cylindrical portion 61a and the inner surface of the cylindrical portion 4.

The distal inner cylindrical portion 61b of the inner cylindrical portion 61 is, for example, a cylindrical male connector portion conforming to ISO 80369-7 issued in 2016. In other words, the connection member 3 includes a male connector portion that defines the flow path 3a therein and is insertable into the female connector portion of the second other medical device.

As shown in Fig. 4 and the like, the outer cylindrical portion 63 includes a substantially cylindrical coupling outer cylindrical portion 63a and a substantially cylindrical distal outer cylindrical portion 63b. The coupling outer cylindrical portion 63a protrudes from an outer edge portion of the annular flange portion 62 to one end side (the right side in Fig. 4) in the axial direction A, and is located around the outer side in the radial direction B of the coupling inner cylindrical portion 61a of the inner cylindrical portion 61. The distal outer cylindrical portion 63b protrudes from the outer edge portion of the annular flange portion 62 to the other end side (the left side in Fig. 4) in the axial direction A, and is located around the outer side in the radial direction B of the distal inner cylindrical portion 61b of the inner cylindrical portion 61.

As shown in Fig. 4 and the like, the coupling outer cylindrical portion 63a of the outer cylindrical portion 63 includes a peripheral wall portion 64 and claw portions 65 that are only partially continuous with the peripheral wall portion 64 and are elastically deformable in the radial direction B at positions other than a continuous position. The coupling outer cylindrical portion 63a covers the outer peripheral surface of the cylindrical portion 4 of the holder member 11.

For the claw portion 65 according to the present embodiment, only one side (the right side in Fig. 4) in the axial direction A is continuous with the peripheral wall portion 64, and the other side in the axial direction A and both sides in the circumferential direction C are not continuous with the peripheral wall portion 64. Therefore, the claw portion 65 according to the present embodiment is elastically deformed and swingable in the radial direction B with a position continuous with the peripheral wall portion 64 on one side in the axial direction A as a fulcrum. As shown in Fig. 1, the plurality of (four in the present embodiment) claw portions 65 according to the present embodiment are provided at different positions in the circumferential direction C.

More specifically, the claw portions 65 according to the present embodiment includes a deformation portion 65a that is elastically deformed in the radial direction B, and a locking protrusion portion 65b that protrudes inward in the radial direction B from a distal portion of the deformation portion 65a.

As described above, the annular groove 4a (see Fig. 4 and the like) extending in the circumferential direction C is formed on the outer surface of the cylindrical portion 4 constituting the second port portion 22b of the holder member 11. The claw portion 65 according to the present embodiment is located on an outer side in the radial direction B of the cylindrical portion 4 of the holder member 11. Then, as shown in Fig. 4, the locking protrusion portion 65b of the claw portion 65 is fitted into the annular groove 4a, and thus the holder member 11 and the connection member 3 are connected in a manner of not being separated from each other in the axial direction A. The claw portion 65 is elastically deformable in the radial direction B as described above. With such a configuration, it is possible to prevent an increase in sliding resistance when the cylindrical portion 4 and the coupling inner cylindrical portion 61a relatively rotate around the central axis O.

As shown in Fig. 4 and the like, female screw portions 63b1 that can be screwed with a lock-type female connector portion conforming to IS080369-7 issued in 2016 are formed on the inner surface of the distal outer cylindrical portion 63b of the outer cylindrical portion 63.

That is, in the connection member 3 according to the present embodiment, the distal inner cylindrical portion 61b and the distal outer cylindrical portion 63b constitute a lock-type male connector portion conforming to ISO 80369-7 issued in 2016. Therefore, the distal inner cylindrical portion 61b and the distal outer cylindrical portion 63b of the connection member 3 according to the present embodiment can be liquid-tightly connected to the lock-type female connector portion conforming to IS080369-7 issued in 2016 by screwing.

More specifically, the distal inner cylindrical portion 61b of the connection member 3 according to the present embodiment is a cylindrical male connector portion conforming to ISO 80369-7 issued in 2016. The distal outer cylindrical portion 63b of the connection member 3 according to the present embodiment is a locking portion that is located on the outer side in the radial direction B of the distal inner cylindrical portion 61b as the male connector portion and can be locked to the second other medical device. The distal outer cylindrical portion 63b as the locking portion is screwed to a female connector portion conforming to ISO 80369-7 issued in 2016 and provided in the second other medical device by the female screw portion 63b1 on the inner surface of the distal outer cylindrical portion 63b, thereby being locked to the second other medical device.

Examples of the material of the connection member 3 according to the present embodiment include materials similar to those usable as the holder member 11 of the device main body 2 described above. However, the connection member 3 is made of a material having a higher flexural modulus than the holder member 11.

### [Connection Structure of Holder Member 11 of Device Main Body 2 and Connection Member 3]

Next, a detail of a connection structure of the holder member 11 of the device main body 2 and the connection member 3 will be described.

The medical device 1 includes two members that are made of materials having different flexural moduli and are connected to each other. Specifically, in the medical device 1 according to the present embodiment, the holder member 11 and the connection member 3 are made of materials having different flexural moduli. The holder member 11 is made of, for example, polypropylene, and the connection member 3 is made of, for example, polycarbonate. In other words, the connection member 3 is a hard member made of a material that is difficult to deform relative to the holder member 11. The holder member 11 is a soft member made of a material that is easily deformed relative to the connection member 3.

The connection member 3 as the hard member is connected to the holder member 11 as the soft member.

Specifically, the cylindrical portion 4 of the holder member 11 and the connection member 3 are restrained from moving in a direction separated from each other along the axial direction A (hereinafter, simply referred to as a "separation direction") . Therefore, even if one of the holder member 11 and the connection member 3 is moved in the separation direction with respect to the other, the holder member 11 and the connection member 3 cannot be separated from each other. That is, the cylindrical portion 4 of the holder member 11 and the connection member 3 are configured not to be separated from each other when moving in the separation direction. In other words, when attention is focused on an optional predetermined portion of the holder member 11 and an optional predetermined portion of the connection member 3, the distance between these two predetermined portions in the axial direction A is set not to be greater than a predetermined distance. Such a configuration can be implemented by arranging the holder member 11 and the connection member 3 in a manner of overlapping each other in the axial direction A and providing the holder member 11 and the connection member 3 with a portion where the holder member 11 and the connection member 3 are in contact with each other and interfere with each other when the holder member 11 and the connection member 3 are separated from each other in the axial direction A.

Specifically, as shown in Figs. 4 and 6, the cylindrical portion 4 of the holder member 11 includes a wall surface 8a that is in contact with and restricts the movement of the connection member 3 in the separation direction. On the other hand, the connection member 3 includes a wall surface 8b that is in contact with the wall surface 8a to restrict the movement of the cylindrical portion 4 in the separation direction.

More specifically, the annular groove 4a is defined on the outer surface of the cylindrical portion 4. The connection member 3 is connected to the cylindrical portion 4 by fitting the locking protrusion portion 65b of the claw portion 65 in the coupling outer cylindrical portion 63a of the connection member 3 into the annular groove 4a of the cylindrical portion 4. That is, since the locking protrusion portion 65b and the annular groove 4a interfere with each other in the axial direction A and come into contact with each other, the connection member 3 and the holder member 11 are not separated from each other in the axial direction A. In other words, in the present embodiment, the above-described wall surface 8a of the cylindrical portion 4 is formed by a groove wall of the annular groove 4a. In the present embodiment, the above-described wall surface 8b of the connection member 3 is formed by an end surface on a distal side of the locking protrusion portion 65b of the claw portion 65.

The cylindrical portion 4 of the holder member 11 and the connection member 3 are also restrained from moving in a reversed direction of the direction separated from each other along the axial direction A (hereinafter, simply referred to as an "approaching direction"). Specifically, as shown in Figs. 4 and 6, the cylindrical portion 4 of the holder member 11 includes a wall surface 9a that contacts and restricts the movement of the connection member 3 in the approaching direction. On the other hand, the connection member 3 includes a wall surface 9b that is in contact with the wall surface 9a to restrict the movement of the cylindrical portion 4 in the approaching direction.

More specifically, when the cylindrical portion 4 of the holder member 11 and the connection member 3 attempt to relatively move in the approaching direction, the distal surface of the cylindrical portion 4 of the holder member 11 and the surface on one side of the annular flange portion 62 of the connection member 3 come into contact with each other. Accordingly, the relative movement of the cylindrical portion 4 of the holder member 11 and the connection member 3 in the approaching direction is restricted. In other words, in the present embodiment, the above-described wall surface 9a of the cylindrical portion 4 is formed by the distal surface of the cylindrical portion 4. In the present embodiment, the above-described wall surface 9b of the connection member 3 is configured with a surface on one side of the annular flange portion 62.

As described above, a relative movement of the cylindrical portion 4 of the holder member 11 and the connection member 3 according to the present embodiment in the separation direction and the approaching direction is restricted.

Further, the cylindrical portion 4 of the holder member 11 as the soft member is supported by the cylindrical support portion 5 of the connection member 3 as the hard member.

Specifically, in the connection member 3 of the present embodiment, the cylindrical support portion 5 includes the coupling inner cylindrical portion 61a as the inner surface support portion 6 that supports the inner surface of the cylindrical portion 4, and the coupling outer cylindrical portion 63a as the outer surface support portion 7 that supports the outer surface of the cylindrical portion 4.

As shown in Fig. 4, the coupling inner cylindrical portion 61a as the inner surface support portion 6 is inserted into the cylindrical portion 4 from a distal opening thereof. As shown in Figs. 4 and 5, the outer surface of the coupling inner cylindrical portion 61a as the inner surface support portion 6 is in contact with the inner surface of the cylindrical portion 4, thereby supporting the inner surface of the cylindrical portion 4 from an inner side toward an outer side in the radial direction.

As shown in Figs. 4 and 5, the inner surface of the coupling outer cylindrical portion 63a as the outer surface support portion 7 is in contact with the outer surface of the cylindrical portion 4, thereby supporting the outer surface of the cylindrical portion 4 from the outer side toward the inner side in the radial direction B.

That is, the cylindrical portion 4 of the holder member 11 according to the present embodiment is sandwiched from both sides of the inner side and the outer side in the radial direction B and held by both sides of the inner side and the outer side in the radial direction B by the coupling inner cylindrical portion 61a as the inner surface support portion 6 of the connection member 3 and the coupling outer cylindrical portion 63a as the outer surface support portion 7 of the connection member 3. More specifically, the cylindrical portion 4 according to the present embodiment is sandwiched and compressed from both the inner side and the outer side in the radial direction B by the coupling inner cylindrical portion 61a as the inner surface support portion 6 of the connection member 3 and the coupling outer cylindrical portion 63a as the outer surface support portion 7 of the connection member 3.

In this way, the holder member 11 and the connection member 3 according to the present embodiment are connected to each other by fitting the annular groove 4a and the claw portion 65 to each other and sandwiching the cylindrical portion 4 from both the inner side and the outer side in the radial direction B by the coupling inner cylindrical portion 61a and the coupling outer cylindrical portion 63a of the connection member 3.

Also, the cylindrical portion 4 of the holder member 11 is supported from both the inner side and the outer side in the radial direction B by the coupling inner cylindrical portion 61a as the inner surface support portion 6 of the connection member 3 and the coupling outer cylindrical portion 63a as the outer surface support portion 7 of the connection member 3, and thus the deformation of the cylindrical portion 4 due to the bending external force can be prevented. That is, even when a bending external force is applied to bend one of the holder member 11 as the soft member and the connection member 3 as the hard member relative to the other, the deformation of the cylindrical portion 4 of the holder member 11 as the soft member is restricted by the cylindrical support portion 5 of the connection member 3. Specifically, the cylindrical portion 4 is restrained from being deformed inward in the radial direction B by coming into contact with the coupling inner cylindrical portion 61a. The cylindrical portion 4 is restrained from being deformed outward in the radial direction B by coming into contact with the coupling outer cylindrical portion 63a. Therefore, the cylindrical portion 4 of the holder member 11 as the soft member is less likely to be deformed by the above-described bending external force, and as a result, it is possible to prevent the cylindrical portion 4 from being deformed and a connection state with the connection member 3 from being released.

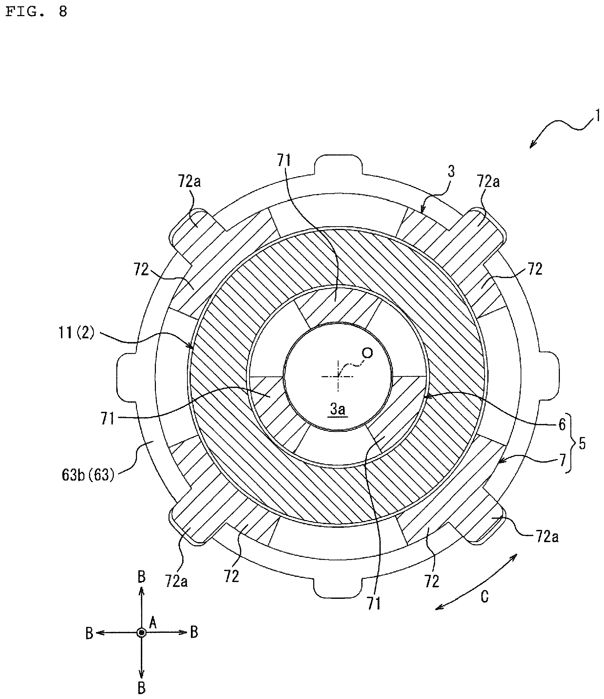

Although the inner surface support portion 6 according to the present embodiment is configured with the coupling inner cylindrical portion 61a, the inner surface support portion 6 is not limited to this configuration. The inner surface support portion 6 is not limited to a cylindrical configuration extending over an entire region in the circumferential direction C, and may be configured with a plurality of portions intermittently arranged at intervals in the circumferential direction C. The interval between two portions adjacent to each other in the circumferential direction C, a circumferential length of each portion, a shape of each portion, and the like can be appropriately designed. The inner surface support portion 6 may be configured with, for example, a cylindrical portion in which one or more opening portions are formed in a peripheral wall. A position, a shape, and the like of the opening portion can be designed as appropriate. A modification of the inner surface support portion 6 will be described in detail later (see Fig. 8).

Although the outer surface support portion 7 according to the present embodiment is configured with the coupling outer cylindrical portion 63a, the outer surface support portion 7 is not limited to this configuration. The outer surface support portion 7 is not limited to a cylindrical configuration extending over an entire region in the circumferential direction C, and may be configured with a plurality of portions intermittently arranged at intervals in the circumferential direction C. An interval between two portions adjacent to each other in the circumferential direction C, a circumferential length of each portion, a shape of each portion, and the like can be appropriately designed. The outer surface support portion 7 may be configured with, for example, a cylindrical portion in which one or more opening portions are formed in a peripheral wall. A position, a shape, and the like of the opening portion can be designed as appropriate. A modification of the outer surface support portion 7 will be described in detail later (see Fig. 8).

In this way, the inner surface support portion 6 and the outer surface support portion 7 are not limited to the configuration of the cylindrical portion. However, as in the present embodiment, it is preferable that the inner surface support portion 6 is configured with an inner cylindrical portion (the coupling inner cylindrical portion 61a in the present embodiment) inserted into the cylindrical portion 4. In this way, it is possible to prevent the ease of deformation of the cylindrical portion 4 toward the inner side in the radial direction B from varying depending on a position in the circumferential direction C. As in the present embodiment, the outer surface support portion 7 is preferably configured with an outer cylindrical portion (the coupling outer cylindrical portion 63a according to the present embodiment) surrounding the outer side in the radial direction B of the cylindrical portion 4. In this way, it is possible to prevent the ease of deformation of the cylindrical portion 4 toward the outer side in the radial direction B from varying depending on the position in the circumferential direction C.

The annular groove 4a and the locking protrusion portion 65b according to the present embodiment are located in a region in the axial direction A in which the inner surface support portion 6 and the outer surface support portion 7 face each other in the radial direction B. In this way, the locking protrusion portion 65b is less likely to fall off from the annular groove 4a. Therefore, it is possible to further prevent the connection state between the device main body 2 and the connection member 3 from being unintentionally released.

Although in the present embodiment, the holder member 11 is provided with the annular groove 4a as a concave portion, and the connection member 3 is provided with the locking protrusion portion 65b as a convex portion to be fitted to the annular groove 4a, the present invention is not limited to this configuration. That is, the holder member 11 may include the convex portion such as the locking protrusion portion, and the concave portion such as the annular groove 4a into which the convex portion is fitted may be provided in the connection member 3.

The connection member 3 according to the present embodiment is rotatably connected to the cylindrical portion 4 constituting the second port portion 22b of the holder member 11. Specifically, the connection member 3 and the holder member 11 are relatively rotatable about the central axis O of the cylindrical portion 4.

More specifically, the coupling outer cylindrical portion 63a of the connection member 3 according to the present embodiment includes the plurality of claw portions 65 disposed at different positions in the circumferential direction C. The locking protrusion portions 65b of the plurality of claw portions 65 are fitted into the annular groove 4a of the cylindrical portion 4. Therefore, when the locking protrusion portion 65b of the claw portion 65 moves in the circumferential direction C in the annular groove 4a, the connection member 3 can rotate around the central axis O of the cylindrical portion 4 while maintaining the connection state with the cylindrical portion 4.

In this way, the medical device 1 includes the two members (the holder member 11 and the connection member 3 according to the present embodiment) that are rotatably connected to each other, and thus, for example, when the medical device 1 is disposed between the plurality of medical tubes on an infusion line, a twist of the infusion line is less likely to occur, and even if the twist occurs, the twist is easily eliminated. Specifically, when the infusion line including a three-way stopcock, which is the medical device 1 according to the present embodiment, is formed, an upstream side portion of the infusion line connected to the first port portion 22a of the holder member 11 is rotatable with respect to a downstream side portion of the infusion line connected to the distal inner cylindrical portion 61b as the male connector portion of the connection member 3. Therefore, the holder member 11 and the connection member 3 relatively rotate, and thus the twisting is less likely to occur between the upstream side portion and the downstream side portion of the infusion line sandwiching the medical device 1. Even if a twist occurs between the upstream side portion and the downstream side portion of the infusion line sandwiching the medical device 1, the twist can be eliminated by relatively rotating the holder member 11 and the connection member 3.

As shown in Fig. 4, in the connection member 3 as the hard member according to the present embodiment, a hollow portion of the distal outer cylindrical portion 63b as the cylindrical male connector portion communicates with the hollow portion of the cylindrical portion 4 of the holder member 11 as the soft member according to the present embodiment. More specifically, in the present embodiment, in a state where the cylindrical portion 4 of the holder member 11 and the connection member 3 are connected to each other, the flow path 3a of the connection member 3 communicates liquid-tightly with the second flow path 52 of the cylindrical portion 4 of the holder member 11. That is, the holder member 11 and the connection member 3 according to the present embodiment are relatively rotatable, and are connected to each other in a manner that the flow path inside the holder member 11 and the connection member 3 is liquid-tight. As described above, a liquid tightness of the flow path can be implemented, for example, by bringing the outer surface of the coupling inner cylindrical portion 61 of the connection member 3 into close contact with the inner surface of the cylindrical portion 4 of the holder member 11.

Further, the medical device 1 according to the present embodiment includes the lock-type female connector portion conforming to ISO 80369-7 issued in 2016, and the lock-type male connector portion conforming to ISO 80369-7 issued in 2016.

Specifically, the cylindrical portion constituting the first port portion 22a is a lock-type female connector portion which includes the male screw portion 22a1 on an outer surface thereof and conforms to ISO 80369-7 issued in 2016. The mixed injection member 13 attached to the third port portion 22c is the lock-type female connector portion which includes the male screw portion 41b on the outer surface thereof and conforms to ISO 80369-7 issued in 2016. Further, in the connection member 3 connecting to the second port portion 22b, the distal inner cylindrical portion 61b and the distal outer cylindrical portion 63b constitute the lock-type male connector portion conforming to ISO 80369-7 issued in 2016.

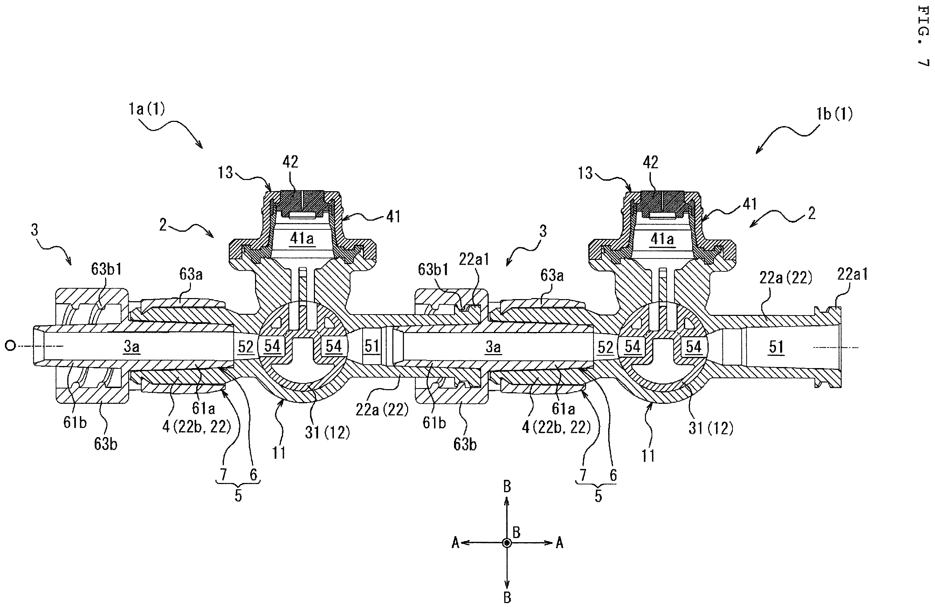

Therefore, in the medical device 1 according to the present embodiment, the medical devices 1 having the same shape can be connected by using the first port portion 22a of the holder member 11 and the connection member 3. Fig. 7 is a cross-sectional view showing a state in which two medical devices 1 are connected to each other by using the first port portion 22a of the holder member 11 and the connection member 3. Each medical device 1 shown in Fig. 7 shows a cross section in the same cross-sectional view as Fig. 4. Hereinafter, for convenience of description, when the two medical devices 1 shown in Fig. 7 are distinguished from each other, the medical device 1 on a left side is referred to as a "first medical device 1a", and the medical device 1 on a right side is referred to as a "second medical device 1b". In Fig. 7, the left side is the flow path downstream side, and the right side is the flow path upstream side.

The first port portion 22a of the first medical device 1a is connected to the distal inner cylindrical portion 61b and the distal outer cylindrical portion 63b constituting the lock-type male connector portion of the connection member 3 of the second medical device 1b. Therefore, specifically, the male screw portion 22a1 of the first port portion 22a of the first medical device 1a is screwed with the female screw portion 63b1 of the connection member 3 of the second medical device 1b. That is, the first medical device 1a and the second medical device 1b are connected by screw joining. Here, the holder member 11 of the first medical device 1a and the holder member 11 of the second medical device 1b are connected to each other via the connection member 3 of the second medical device 1b. Therefore, even when the holder member 11 of one of the first medical device 1a and the second medical device 1b rotates relative to the other holder member 11 in a direction in which the above-described screw joint is loosened, the torque is used for rotation between the holder member 11 and the connection member 3 in the second medical device 1b. That is, the above-described torque makes it difficult for the screw joint between the holder member 11 of the first medical device 1a and the connection member 3 of the second medical device 1b to loosen.

As described above, the second medical device 1b according to the present embodiment includes the holder member 11 and the connection member 3 as the two rotatably connected members. The connection member 3 of the second medical device 1b can be screw-joined to the holder member 11 of the first medical device 1a having the same shape as the second medical device 1b. With such a configuration, the plurality of medical devices 1 can be connected in series by screw joining. Even if an external force acts on the holder member 11 of the first medical device 1a in a direction in which the screw joint with the connection member 3 of the second medical device 1b is loosened, the torque is easily used to rotate the holder member 11 and the connection member 3 of the second medical device 1b. Further, even if an external force acts on the holder member 11 of the second medical device 1b in a direction in which the screw joint between the holder member 11 of the first medical device 1a and the connection member 3 of the second medical device 1b is loosened, the torque is easily used to rotate the holder member 11 and the connection member 3 of the second medical device 1b. In this way, the screw joint between the holder member 11 of the first medical device 1a and the connection member 3 of the second medical device 1b is less likely to be loosened. That is, in a state where the plurality of medical devices 1 are connected by screw joining, it is possible to prevent loosening of the screw joining or release of the screw joining due to an unintended external force or the like acting on the holder member 11.

Next, a modification of the cylindrical support portion 5 in the connection member 3 as the hard member will be described with reference to Fig. 8. Fig. 8 shows a cross-sectional view taken along the same cross section as Fig. 5. As shown in Fig. 8, the cylindrical support portion 5 includes the inner surface support portion 6 and the outer surface support portion 7.

The inner surface support portion 6 shown in Fig. 8 is configured with a plurality of curved plate portions 71 arranged at intervals in the circumferential direction C. Three curved plate portions 71 shown in Fig. 8 are arranged at intervals in the circumferential direction C.

The outer surface support portion 7 shown in Fig. 8 is configured with a plurality of curved plate portions 72 arranged at intervals in the circumferential direction C. Four curved plate portions 72 shown in Fig. 8 are arranged at intervals in the circumferential direction C.

In this way, the inner surface support portion 6 and the outer surface support portion 7 of the cylindrical support portion 5 may not be cylindrical portions.

The number, a shape, and a circumferential length of the curved plate portions 71 constituting the inner surface support portion 6 are not particularly limited. However, in an optional cross section of the cylindrical portion 4 orthogonal to the axial direction A, a sum of circumferential lengths of convex curved surfaces of all curved plate portions 71 that are in contact with the inner surface of the cylindrical portion 4 is preferably 1/3 or more of an inner circumferential length of the cylindrical portion 4 (120° or more at a center angle), and more preferably 2/3 or more of the inner circumferential length of the cylindrical portion 4 (240° or more at the center angle). Further, it is preferable that the convex curved surfaces of the curved plate portions 71 which are brought into contact with the inner surface of the cylindrical portion 4 are dispersed over the entire region in the circumferential direction C at predetermined intervals. In this way, the inner surface of the cylindrical portion 4 is supported by the curved plate portions 71 as the inner surface support portion 6 over the entire region in the circumferential direction C. Therefore, it is possible to prevent the ease of deformation of the cylindrical portion 4 toward the inner side in the radial direction B from varying depending on a position in the circumferential direction C.

Further, the number, a shape, and a circumferential length of the curved plate portions 72 constituting the outer surface support portion 7 are not particularly limited. However, in an optional cross section of the cylindrical portion 4 orthogonal to the axial direction A, a sum of circumferential lengths of concave curved surfaces of all of the curved plate portions 72 that are in contact with the outer surface of the cylindrical portion 4 is preferably 1/3 or more of an outer circumferential length of the cylindrical portion 4 (120° or more at a center angle), and more preferably 2/3 or more of the outer circumferential length of the cylindrical portion 4 (240° or more at the center angle). Further, it is preferable that the concave curved surfaces of the curved plate portions 72 which are brought into contact with the outer surface of the cylindrical portion 4 are dispersed over the entire region in the circumferential direction C at predetermined intervals. In this way, the outer surface of the cylindrical portion 4 is supported by the curved plate portions 72 as the outer surface support portion 7 over the entire region in the circumferential direction C. Therefore, it is possible to prevent the ease of deformation of the cylindrical portion 4 toward the outer side in the radial direction B from varying depending on a position in the circumferential direction C.

It is preferable that the curved plate portion 71 constituting the inner surface support portion 6 and the curved plate portion 72 constituting the outer surface support portion 7 overlap each other in the radial direction B in at least a part of the circumferential direction C. In the example shown in Fig. 8, each of the curved plate portions 71 constituting the inner surface support portion 6 overlaps at least one of the curved plate portions 72 constituting the outer surface support portion 7 in at least a part of the circumferential direction C in the radial direction B. In this way, it is possible to further prevent deformation of the inner side and the outer side of the cylindrical portion 4 in the radial direction B due to the bending external force. In particular, it is preferable that all regions in the circumferential direction of the curved plate portions 71 constituting the inner surface support portion 6 overlap the curved plate portions 72 constituting the outer surface support portion 7 in the radial direction B. In other words, it is preferable that the curved plate portions 72 constituting the outer surface support portion 7 completely cover the outer side in the radial direction B of each of the curved plate portions 71 constituting the inner surface support portion 6. In this way, it is possible to further prevent the deformation of the inner side and the outer side of the cylindrical portion 4 in the radial direction B due to the bending external force.

The curved plate portion 72 as the outer surface support portion 7 shown in Fig. 8 includes a rib 72a protruding outward in the radial direction B. By forming the curved plate portion 72 including the rib 72a as described above, the strength of the curved plate portion 72 can be increased, and the deformation of the cylindrical portion 4 toward the outer side in the radial direction B can be further prevented.

Finally, an infusion set 100 including the medical device 1 shown in Figs. 1 to 7 will be described by way of example with reference to Fig. 9. Fig. 9 is a diagram showing the infusion set 100. The infusion set 100 shown in Fig. 9 includes the three-way stopcock as the medical device 1 shown in Figs. 1 to 7, but may include a medical connector having another shape as the medical device according to the present disclosure. The infusion set may include the medical device including the cylindrical support portion 5 shown in Fig. 8.

The infusion set 100 is formed by an infusion line connecting an infusion bag (not shown in Fig. 9) to an indwelling needle (also not shown in Fig. 9). Specifically, the infusion set 100 includes a plurality of medical tubes 111, an drip chamber 112 capable of visually recognizing a flow rate of an infusion agent supplied from the infusion bag, an adjustment clamp 113 that adjusts the flow rate of the infusion agent in the medical tube 111, an air vent filter 114 that discharges (or supplies) air present in the infusion line, and a one-touch clamp 115 that closes the medical tube 111.

In the infusion line of the infusion set 100 shown in Fig. 9, the three-way stopcock as the medical device 1 is provided between the adjustment clamp 113 and the air vent filter 114. The three-way stopcock as the medical device 1 connects a first medical tube 111a extending from the drip chamber 112 to a downstream side and a second medical tube 111b extending to an upstream side of the air vent filter 114, and constitutes a part of a main line of the infusion line. More specifically, the first port portion 22a of the three-way stopcock as the medical device 1 is connected to the first medical tube 111a as the first other medical device. The lock-type male connector portion conforming to ISO 80369-7 issued in 2016 is provided at a distal portion of the first medical tube 111a. The connection member 3 connected to the second port portion 22b of the three-way stopcock as the medical device 1 is connected to the second medical tube 111b as the second other medical device. The lock-type female connector portion conforming to ISO 80369-7 issued in 2016 is provided at a proximal end portion of the second medical tube 111b.

A third medical tube 111c as the third other medical device is connected to the third port portion 22c of the three-way stopcock as the medical device 1 via the mixed injection member 13 (see Fig. 1). The lock-type male connector portion conforming to ISO 80369-7 issued in 2016 is provided at a distal portion of the third medical tube 111c. The third medical tube 111c connected to the third port portion 22c via the mixed injection member 13 (see Fig. 1) constitutes a subline of the infusion line. Therefore, the main line and the subline of the infusion line are connected to each other inside the three-way stopcock as the medical device 1.

By rotating the handle portion 32 of the three-way stopcock as the medical device 1, it is possible to switch a connection relationship between the first flow path 51 (see Fig. 4) defined by the first port portion 22a, the second flow path 52 (see Fig. 4) defined by the second port portion 22b, and the third flow path 53 (see Fig. 4) defined by the third port portion 22c.

Although in the present embodiment, the three-way stopcock as the medical device 1 is provided between the adjustment clamp 113 and the air vent filter 114, a position of the medical device 1 is not limited to this position, and can be provided at a desired position. Although the infusion set 100 shown in Fig. 9 includes only one three-way stopcock as the medical device 1, as shown in Fig. 7, a plurality of three-way stopcocks as the medical device 1 may be continuously connected.

The medical device according to the present disclosure is not limited to a specific configuration specified in the embodiment and the modification described above, and various modifications and changes can be made without departing from a gist of the invention described in the claims. Specifically, although the medical device 1 described above is a three-way stopcock, the medical device 1 may be another medical device such as a medical connector having another shape. Therefore, the two members made of materials having different flexural moduli and connected to each other are not limited to the holder member 11 and the connection member 3 described above, and may be appropriately determined according to the specific configuration of the medical device.

### Industrial Applicability

The present disclosure relates to a medical device.

### Reference Signs List

1: medical device
1a: first medical device
1b: second medical device
2: device main body
3: connection member
3a: flow path (hollow portion)
4: cylindrical portion
4a: annular groove
4b: insertion restricting portion
5: cylindrical support portion
6: inner surface support portion
7: outer surface support portion
8a, 8b, 9a, 9b: wall surface
11: holder member
12: cock member
13: mixed injection member
21: holder main body
22: port portion
22a: first port portion
22a1: male screw portion
22b: second port portion
22c: third port portion
31: cock main body
32: handle portion
41: cap member
41a: insertion opening
41b: male screw portion
42: elastic valve body
42a: slit
43: top surface cap
44: bottom surface cap
50: hollow portion
51: first flow path
52: second flow path
53: third flow path
54: connection flow path
61: inner cylindrical portion
61a: coupling inner cylindrical portion
61b: distal inner cylindrical portion
62: annular flange portion
63: outer cylindrical portion
63a: coupling outer cylindrical portion
63b: distal outer cylindrical portion
63b1: female screw portion
64: peripheral wall portion
65: claw portion
65a: deformation portion
65b: locking protrusion portion
71: curved plate portion
72: curved plate portion
72a: rib
81: proximal inner peripheral surface of cylindrical portion of holder member
82: distal inner peripheral surface of cylindrical portion of holder member
83: intermediate inner peripheral surface of cylindrical portion of holder member
84: proximal outer peripheral surface of coupling inner cylindrical portion of connection member
85: distal outer peripheral surface of coupling inner cylindrical portion of connection member
85a: distal root portion surface
85b: distal portion surface
100: infusion set
111: medical tube
111a: first medical tube
111b: second medical tube
111c: third medical tube
112: drip chamber
113: adjustment clamp
114: air vent filter
115: one-touch clamp
A: axial direction of cylindrical portion of holder member and inner cylindrical portion of connection member
B: radial direction of cylindrical portion of holder member and inner cylindrical portion of connection member
C: circumferential direction of cylindrical portion of holder member and inner cylindrical portion of connection member
O: central axis of cylindrical portion of holder member
T1: insertion region
T2: contact region

## Claims

1. A medical device, comprising:
two members that are made of materials having different flexural moduli and are connected to each other, wherein
a soft member made of a material having a low flexural modulus of the two members includes a cylindrical portion,
a hard member made of a material having a high flexural modulus of the two members includes a cylindrical support portion that supports the cylindrical portion, and
the cylindrical support portion includes an inner surface support portion that supports an inner surface of the cylindrical portion, and an outer surface support portion that supports an outer surface of the cylindrical portion.

2. The medical device according to claim 1, wherein
the soft member and the hard member are rotatably connected to each other.

3. The medical device according to claim 1 or 2, wherein the hard member includes a male connector portion that can be inserted into a female connector portion of another medical device.

4. The medical device according to claim 3, wherein
a hollow portion of the male connector portion of the hard member communicates with a hollow portion of the cylindrical portion of the soft member.

5. The medical device according to claim 3 or 4, wherein the hard member includes a locking portion that is located on a radially outer side of the male connector portion and can be locked to another medical device.

6. The medical device according to claim 5, wherein
the locking portion is a locking cylindrical portion in which a female screw portion capable of being screwed to another medical device is formed on an inner surface.

7. The medical device according to any one of claims 3 to 6, wherein
the soft member includes a female connector portion into which a male connector portion of still another medical device is inserted.

8. The medical device according to any one of claims 1 to 7, wherein
the inner surface support portion is an inner cylindrical portion inserted into the cylindrical portion, and
an outer surface of the inner cylindrical portion supports an inner surface of the cylindrical portion.

9. The medical device according to any one of claims 1 to 8, wherein
the outer surface support portion is an outer cylindrical portion surrounding a radially outer side of the cylindrical portion, and
an inner surface of the outer cylindrical portion supports an outer surface of the cylindrical portion.
